# EUROPEAN PATENT APPLICATION

(11) **EP 4 194 466 A1**
(43) Date of publication of application: **14.06.2023**
(21) Application number: 21854000.3
(22) Date of filing: 09.08.2021
(51) Int. Cl.: C07K 16/10, A61P 31/14, A23L 33/18, A61K 39/00

(54) **ANTIVIRAL COMPOSITION AGAINST CORONAVIRUS**

(30) Priority: 07.08.2020 US 202063062654 P
(71) Applicant: Novelgen Co., Ltd., Suwon-si, Gyeonggi-do 16230 (KR)
(72) Inventor: KIM, Tai Hyun, Suwon-si, Gyeonggi-do 16420 (KR); OH, Kwang Ji, Daejeon 34010 (KR); KIM, Won Keun, Chuncheon-si, Gangwon-do 24415 (KR)
(74) Representative: dompatent von Kreisler Selting Werner - Partnerschaft von Patent- und Rechtsanwälten mbB
(86) International application number: PCT/KR2021/010460
(87) International publication number: WO 2022/031137

(57) **Abstract**

The present invention provides an antiviral composition for coronavirus, and more particularly, an antiviral composition, including an antibody or fragment thereof, which includes: a heavy chain CDR1 (VH CDR1) represented by SEQ ID NO: 1; VH CDR2 represented by SEQ ID NO: 2; VH CDR3 represented by SEQ ID NO: 3; a light chain CDR1 (VL CDR1) represented by SEQ ID NO: 4; VL CDR2 represented by SEQ ID NO: 5; and VL CDR3 represented by SEQ ID NO: 6, so as to exhibit excellent antiviral effects to coronavirus, while achieving effects of preventing, treating or improving diseases derived from coronavirus.

## Description

### [Technical Field]

The present invention relates to an antiviral composition for coronavirus.

### [Background Art]

Coronavirus refers to viruses belonging to the family Coronaviridae and is generally found in a variety of mammals, including birds and humans. Coronaviruses are known to cause respiratory and/or digestive infections depending on the nature and host of the virus.

Viruses belonging to the coronavirus family generally have a single-stranded positive sense RNA genome with a length of 27-32 kb, in which a cap is present at the 5th end of the genome, and a poly A tail is present at the 3rd end. A diameter of the coronavirus is about 80 to 200 nm, and club-shaped spikes are present on the surface of the virus particles. The virus has an envelope, and major outer membrane proteins such as spike proteins and envelope proteins also exist. The spike protein is involved in viral infection and pathogenicity, including induction of neutralizing antibody, receptor binding, membrane fusion, etc. The envelope protein plays a role of protecting viruses when forming viral particles or when moving between cells of a host after viral infection.

Coronaviruses are classified into four genera: Alpha, Beta, Gamma, and Delta. The alpha-coronavirus genus is further divided into types 1a and 1b, while the beta-coronavirus genus is divided into types 2a, 2b, 2c, and 2d. The alpha-coronavirus and beta-coronavirus genera are known to infect humans and animals, while the gamma-coronavirus and delta-coronavirus genera are known to infect animals.

Coronaviruses have been commonly known as an etiologic agent to derive common cold symptoms in humans, but are recently attracting attention as a major etiologic agent of new infectious diseases that occur in humans such as Severe Acute Respiratory Syndrome (SARS), Middle East Respiratory Syndrome (MERS) and new infectious pneumonia caused by Coronavirus Infectious Disease-19 (COVID-19).

### [Summary of Invention]

### [Problems to be Solved by Invention]

An object of the present invention is to provide an antiviral composition for coronavirus.

In addition, another object of the present invention is to provide a pharmaceutical composition for prevention or treatment of coronavirus-infectious diseases (COVID).

Further, another object of the present invention is to provide a food composition for prevention or improvement of coronavirus-infectious diseases (COVID).

### [Means for Solving Problems]

1. An antiviral composition for coronavirus, including an antibody or fragment thereof, which includes: a heavy chain CDR1 (VH CDR1) represented by SEQ ID NO: 1; VH CDR2 represented by SEQ ID NO: 2; VH CDR3 represented by SEQ ID NO: 3; a light chain CDR1 (VL CDR1) represented by SEQ ID NO: 4; VL CDR2 represented by SEQ ID NO: 5; and VL CDR3 represented by SEQ ID NO: 6.
2. The antiviral composition according to the above 1, wherein the antibody or fragment thereof includes a heavy chain variable region including an amino acid sequence of SEQ ID NO: 7.
3. The antiviral composition according to the above 1, wherein the antibody or fragment thereof includes a light chain variable region including an amino acid sequence of SEQ ID NO: 8.
4. The antiviral composition according to the above 1, wherein the fragment is at least one selected from the group consisting of single-chain antibodies, Fv, scFv, di-scFv, Fab, Fab', F(ab')₂ and diabody.
5. The antiviral composition according to the above 1, wherein the fragment is scFv including an amino acid sequence of SEQ ID NO: 9.
6. The antiviral composition according to the above 1, wherein the antibody or fragment thereof is a humanized antibody or fragment thereof; or a chimeric antibody or fragment thereof.
7. The antiviral composition according to the above 1, wherein the coronavirus is at least one selected from the group consisting of genus alpha-coronavirus or genus beta-coronavirus.
8. The antiviral composition according to the above 1, wherein the coronavirus is at least one selected from the group consisting of SARS-coronavirus-2 (SARS-CoV-2), human coronavirus OC43 (hCoV-OC43) and pig epidemic diarrhea virus (PEDV).
9. A pharmaceutical composition for prevention or treatment of a coronavirus-infectious disease, including the composition of any one of the above 1 to 8.
10. The pharmaceutical composition according to the above 9, wherein the coronavirus is at least one selected from the group consisting of SARS-coronavirus-2 (SARS-CoV-2), human coronavirus OC43 (hCoV-OC43) and pig epidemic diarrhea virus (PEDV).
11. A food composition for prevention or treatment of coronavirus-infectious diseases, including the composition of any one of the above 1 to 8.
12. The food composition according to the above 11, wherein the coronavirus is at least one selected from the group consisting of SARS-coronavirus-2 (SARS-CoV-2), human coronavirus OC43 (hCoV-OC43) and pig epidemic diarrhea virus (PEDV).

### [Advantageous effects]

The antiviral composition of the present invention includes an antibody or a fragment thereof having activity for inhibiting infection and proliferation of coronavirus thus to exhibit excellent antiviral effects on coronavirus, while achieving preventive, therapeutic or improvement effects on COVIDs.

### [Brief Description of Drawings]

FIG. 1 illustrates a sequence of an antibody or fragment thereof according to an embodiment of the present invention.
FIG. 2 illustrates results of confirming intracellular penetration activity in an embodiment of the present invention.
FIG. 3 illustrates results of confirming efficacy of hydrolyzing viral genes in an embodiment of the present invention.
FIG. 4 illustrates results of confirming intracellular toxicity to concentration through MTT analysis in an embodiment of the present invention.
FIG. 5 illustrates results of confirming intracellular toxicity to concentration through NGS analysis in an embodiment of the present invention.
FIG. 6 illustrates results of confirming antiviral efficacy against SARS-CoV-2 through qReal-time PCR in an embodiment of the present invention.
FIG. 7 illustrates results of confirming antiviral efficacy against SARS-CoV-2 through qReal-time PCR in an embodiment of the present invention.
FIG. 8 illustrates results of confirming antiviral efficacy against SARS-CoV-2 to concentration through real-time PCR in an embodiment of the present invention.
FIG. 9 illustrates results of confirming antiviral efficacy against SARS-CoV-2 through plaque assay in an embodiment of the present invention.
FIG. 10 illustrates results of confirming antiviral efficacy against hCoV-OC43 and PEDV through qReal-time PCR in an embodiment of the present invention.
FIG. 11 illustrates results of confirming antiviral efficacy against hCoV-OC43 and PEDV to concentration through Real-time PCR in an embodiment of the present invention.
FIG. 12 illustrates results of confirming antiviral efficacy against hCoV-OC43 and PEDV through plaque assay in an embodiment of the present invention.
FIG. 13 illustrates results of confirming antiviral efficacy against hCoV-OC43 and PEDV through Immunoblot in an embodiment of the present invention.

### [Mode for Carrying out Invention]

Hereinafter, the present invention will be described in detail.

The present invention provides an antiviral composition for coronavirus, including an antibody or fragment thereof which includes: a heavy chain CDR1 (VH CDR1) represented by SEQ ID NO: 1; VH CDR2 represented by SEQ ID NO: 2; VH CDR3 represented by SEQ ID NO: 3; a light chain CDR1 (VL CDR1) represented by SEQ ID NO: 4; VL CDR2 represented by SEQ ID NO: 5; and VL CDR3 represented by SEQ ID NO: 6.

The term "complementarity determining region (CDR)" refers to a region involved in antigen recognition, wherein the specificity of the antibody to an antigen is determined when a sequence of this region changes. As used herein, the VH CDR refers to a heavy chain complementarity determining region present in a heavy chain variable region, and the VL CDR refers to a light chain complementarity determining region present in a light chain variable region.

The term "variable region" refers to a region that shows sequence variation while performing a function of specifically binding to an antigen, and CDR1, CDR2 and CDR3 exist in the variable region. A framework region (FR) portion exists between the CDRs, which may serve to support the CDRs. Irrespective of the framework regions, CDRs may be important regions that allow an antibody or fragment thereof to exhibit specific functions.

The antibody or fragment thereof may include the heavy chain variable region which includes: a heavy chain CDR1 (VH CDR1) represented by SEQ ID NO: 1, VH CDR2 represented by SEQ ID NO: 2, and VH CDR3 represented by SEQ ID NO: 3; and a light chain variable region which includes: a light chain CDR1 (VL CDR1) represented by SEQ ID NO: 4, VL CDR2 represented by SEQ ID NO: 5, and VL CDR3 represented by SEQ ID NO: 6.

The antibody or fragment thereof may include a heavy chain variable region which includes an amino acid sequence of SEQ ID NO: 7. According to one embodiment, the antibody or fragment thereof may include a heavy chain variable region consisting of the amino acid sequence of SEQ ID NO: 7.

The antibody or fragment thereof may include a light chain variable region which includes an amino acid sequence of SEQ ID NO: 8. According to one embodiment, the antibody or fragment thereof may include a light chain variable region consisting of the amino acid sequence of SEQ ID NO: 8.

A fragment of antibody ('antibody fragment') may be at least one selected from the group consisting of single chain antibodies, Fv, scFv, di-scFv, Fab, Fab', F(ab')₂, Fd, LC, dAb, CDR, scFv-Fc, and diabodies.

The antibody fragment may include an amino acid sequence of SEQ ID NO: 9. According to one embodiment, the antibody fragment may consist of the amino acid sequence of SEQ ID NO: 9. According to one embodiment, the antibody fragment may consist of scFv including the amino acid sequence of SEQ ID NO: 9.

An antibody or fragment thereof may be a humanized antibody or fragment thereof; a chimeric antibody or fragment thereof. Humanized antibodies may be prepared by methods known in the art.

According to an embodiment, the antibody fragment may be a humanized scFv including CDR sequences of the scFv of SEQ ID NO: 9.

The antibody or fragment thereof according to the present invention may have nuclease activity and can degrade RNA and DNA of viruses. The antibody or fragment thereof may penetrate into the cytoplasm of a virus-infected host cell without further treatment. The penetrating effect of the antibody or fragment thereof into the cytoplasm may be considered to be due to Caveolae mediated endocytosis.

The antibody or fragment thereof according to the present invention may penetrate into the cytoplasm of a host infected with coronavirus and, if removing naked viral RNA from the infected cells, it may induce a decrease in the amount of viral gene replication and a decrease in viral proteins, while improving the efficacy of virus treatment.

The coronavirus may be at least one selected from the group consisting of genus alpha-coronavirus, genus beta-coronavirus, genus gamma-coronavirus, and genus delta-coronavirus.

The coronavirus may be at least one selected from the group consisting of the genus alpha-coronavirus or the genus beta-coronavirus.

The coronavirus may be a human or non-human animal coronavirus, for example, the animal may be a pig, a cow or the like.

The coronaviruses may be at least one selected from the group consisting of canine coronavirus (CCoV), feline coronavirus (FeCoV), pig epidemic diarrhea virus (PEDV), mouse hepatitis virus (MHV), turkey coronavirus (TCoV), transmissible gastrointestinal virus (TGEV), rat coronavirus (RCV), human coronavirus 229E (HCoV-229E), human coronavirus NL63 (NL), human coronavirus OC43 (HCoV-OC43), SARS coronavirus (SARS-CoV), Middle East respiratory syndrome coronavirus (MERS-Cov) and SARS coronavirus 2 (SARS-CoV-2).

According to one embodiment, the coronavirus may be at least one selected from the group consisting of SARS-coronavirus-2 (SARS-CoV-2), human coronavirus OC43 (hCoV-OC43) and pig epidemic diarrhea virus (PEDV).

Further, the present invention provides a pharmaceutical composition for prevention or treatment of a coronavirus infectious disease, which includes the above-described antiviral composition.

Since antiviral compositions, antibodies or fragments thereof, and coronaviruses have already been described above, and therefore will not be described in detail.

The term "prevention" refers to any action that suppresses a disease caused by a virus or delays the onset of the disease.

The term "treatment" refers to any action that improves or beneficially changes symptoms of a disease caused by a virus.

The coronaviruses may be selected from the group consisting of canine coronavirus (CCoV), feline coronavirus (FeCoV), pig epidemic diarrhea virus (PEDV), mouse hepatitis virus (MHV), turkey coronavirus (TCoV), transmissible gastrointestinal virus (TGEV), rat coronavirus (RCV), human coronavirus 229E (HCoV-229E), human coronavirus NL63 (NL), human coronavirus OC43 (HCoV-OC43), SARS coronavirus (SARS-CoV), Middle East respiratory syndrome coronavirus (MERS-Cov) and SARS-CoV-2 (SARS-CoV-2).

The coronavirus-infectious disease may include flu, cold, sore throat, bronchitis or pneumonia, but they are not limited thereto so long as they are diseases caused by viral infection. The coronavirus infectious disease may be Coronavirus Infectious Disease-19 (COVID-19).

The pharmaceutical composition of the present invention may be used alone or in combination with methods using surgery, drug therapy and biological response regulators for prevention and treatment of diseases.

The pharmaceutical composition may include additional ingredients having a therapeutic effect on viral infection, and the additional ingredient may be any component known to have therapeutic effects on viral infection, including compounds, natural products, proteins, peptides, nucleic acids and the like.

The pharmaceutical composition may further include suitable carriers, excipients and diluents commonly used in the preparation of the composition. Such carriers, excipients and diluents may include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil or the like.

The pharmaceutical composition of the present invention may be formulated in oral dosage forms such as powders, granules, tablets, capsules, suspensions, emulsions, syrups, aerosols, external preparations, suppositories, and sterile injection solutions according to conventional methods, and such formulations may be performed by a method commonly performed in the pharmaceutical field. In the case of formulation, it may be prepared by additionally applying diluents or excipients such as commonly used fillers, extenders, binders, wetting agents, disintegrants, and surfactants.

Solid preparations for oral administration may include tablets, pills, powders, granules, capsules, etc., and such solid preparations may include at least one excipient, for example, starch, calcium carbonate, sucrose or lactose, gelatin, etc. In addition to simple excipients, lubricants such as magnesium stearate and talc are also used. Liquid formulations for oral administration may include suspensions, internal solutions, emulsions, syrups, etc., and various excipients such as wetting agents, sweeteners, fragrances, and preservatives, other than water and liquid paraffin, which are commonly used simple diluents, may be included. Formulations for parenteral administration may include sterile aqueous solutions, non-aqueous solutions, suspensions, emulsions, freeze-dried preparations and suppositories. As the non-aqueous agent and suspending agent, propylene glycol, polyethylene glycol, vegetable oils such as olive oil, and injectable esters such as ethyl oleate may be used. As the base of the suppository, witepsol, macrogol, tween, cacao butter, laurin, glycerogelatin, etc. may be used.

The pharmaceutical composition of the present invention may be appropriately administered according to the patient's condition and body weight, the degree of disease, drug form, administration route and period. For example, the pharmaceutical composition of the present invention may be administered at about 0.1 to 1,000 mg/kg per day, and specifically 100 to 300 mg/kg.

The concentration of the antibody or fragment thereof included in the pharmaceutical composition of the present invention may be less than 40 µM, less than 39 µM, less than 38 µM, less than 37 µM, less than 36 µM, less than 35 µM, less than 34 µM, less than 33 µM, less than 32 µM, less than 31 µM, less than 30 µM, less than 29 µM, less than 28 µM, less than 27 µM, less than 26 µM, less than 25 µM, less than 24 µM, less than 23 µM, less than 22 µM, less than 21 µM, less than 20 µM, less than 19 µM, less than 18 µM, less than 17 µM, less than 16 µM, less than 15 µM, less than 14 µM, less than 13 µM, less than 12 µM, less than 11 µM, or less than 10 µM. The concentration of the antibody or fragment thereof included in the pharmaceutical composition of the present invention may exceed 0 µM.

Further, the present invention provides a food composition for prevention or improvement of coronavirus infectious disease, which includes the above-described antiviral composition.

The antiviral compositions, antibodies or fragments thereof, coronaviruses and diseases have been described above, and therefore will not be described in detail.

The term "improvement" refers to any action that reduces the symptoms of a disease caused by a virus.

The food composition may be used together with other foods or food ingredients, and may be appropriately used according to a conventional method.

A mixing amount of the active ingredient included in the food composition may be suitably determined according to the purpose of its use (for prevention or improvement). Specifically, the concentration of the antibody or fragment thereof included in the food composition may be less than 40 µM, less than 39 µM, less than 38 µM, less than 37 µM, less than 36 µM, less than 35 µM, less than 34 µM, less than 33 µM, less than 32 µM, less than 31 µM, less than 30 µM, less than 29 µM , less than 28 µM, less than 27 µM, less than 26 µM, less than 25 µM, less than 24 µM, less than 23 µM, less than 22 µM, less than 21 µM, less than 20 µM, less than 19 µM, less than 18 µM, less than 17 µM, less than 16 µM, less than 15 µM, less than 14 µM, less than 13 µM, 12 µM, less than 11 µM or less than 10 µM. The concentration of the antibody or fragment thereof included in the food composition of the present invention may exceed 0 µM.

The type of food composition is not particularly limited and may include, for example, meat, sausage, bread, chocolate, candy, snacks, confectionery, pizza, ramen, other noodles, gums, dairy products including ice cream, various soups, beverages, tea, drinks, alcoholic beverages and vitamin complexes.

Like conventional foods, the food composition may further include additional ingredients such as flavoring agents or natural carbohydrates other than active ingredients. The natural carbohydrates may include: monosaccharides such as glucose and fructose; disaccharides such as maltose and sucrose; polysaccharides such as dextrin and cyclodextrin; as well as a sugar alcohol such as xylitol, sorbitol, or erythritol. Examples of the sweetener may include natural sweeteners such as taumatin and stevia extract; and synthetic sweeteners such as saccharin and aspartame.

In addition to the above, the health food of the present invention may include various nutrients, vitamins, electrolytes, flavoring agents, colorants, pectic acid and its salts, alginic acid and its salts, organic acids, protective colloidal thickeners, pH adjusters, stabilizers, preservatives, glycerin, alcohol, carbonation agents used in carbonated beverages and the like. In addition, it may contain the pulp for production of natural fruit juice, fruit juice beverage and vegetable beverage.

Further, the present invention provides a method for preventing or treating a coronavirus infectious disease, which includes administering the above-described antiviral composition to a subject.

The subject may be a human and/or a non-human animal, and specifically, a mammal capable of exhibiting beneficial effects by treatment using the drug of the present invention.

The subject may include all subjects who have or are at risk of having symptoms of a coronavirus-infectious disease (e.g., fever, malaise, cough, dyspnea, pneumonia, sputum, sore throat, headache, hemoptysis, nausea or diarrhea). The coronavirus is the same as described above, and therefore will not be described in detail.

An amount effective for preventing or treating a coronavirus-infectious disease means an amount capable of achieving a desired outcome in a subject in need thereof, or an amount capable of providing objective or subjective benefits to a subject in need thereof.

The amount effective for preventing or treating a coronavirus-infectious disease may be single or multiple doses. An effective amount for preventing or treating a coronavirus-infectious disease may be an amount when the drug of the present invention is provided alone, but may also be an amount when provided in combination with one or more other compositions (e.g., other respiratory disease treatment agents, etc.).

Numerical values described herein should be construed to include equivalent ranges unless otherwise specified.

Further, the present invention provides the use of a pharmaceutical composition which includes the above-described antiviral composition for prevention or treatment of a coronavirus-infectious disease.

Hereinafter, examples will be given to describe the present invention in detail.

### 1. Preparation of antibody or fragment thereof

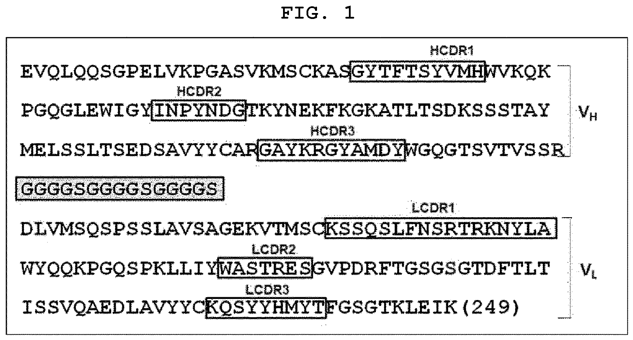

Using *E. coli* BL21 cells as a host, a recombinant protein NVG308 (scFv) having the amino acid sequence of SEQ ID NO: 9 of Table 1 below was produced (see FIG. 1). Table 1 below listed NVG308-related sequences (scFv sequences; VH, VL, CDR sequences thereof; and gene sequences thereof).

The plasmid containing the nucleotide sequence of SEQ ID NO: 10 in *E. coli* BL21 cells treated with CaCl₂ was subjected to heat treatment at 42 °C for 1 minute and then stabilized at 37 °C for 1 hour. Thereafter, colonies transformed by spreading the stabilized product on a selective medium containing antibiotics were selected, and cells were cultured thereon to express scFv. In order to facilitate the adaptation of *E. coli* BL21 cells to the incubator, the cells banked at -80 °C in a concentration of 20% glycerol were pre-cultured for 8 to 9 hours in 30 ml of LB broth medium. The cell concentration was (A600 nm) OD 5 to 7 at this time. In this culture, LB north medium was used, and a volume of the culture medium was 3L. Prior to inoculation of the pre-cultured seeds into the main culture, selection markers, that is, kanamycin and chloramphenicol were added to reach 50 mg/ml and 25 mg/ml, respectively. Next, 30 ml of the prepared seeds was inoculated to maintain an inoculation amount of 1% relative to a volume of the main culture solution. After seed inoculation, the temperature was maintained at 37 °C, and pH was maintained at 6.8 using ammonia water having a concentration of 25 to 30%. Further, in order to maintain an aerobic environment, DO was set to be 40% or more. 3 to 4 hours after seed inoculation, when the cell concentration (A600 nm) OD reached 0.7 to 0.9, induction was started with 1 mM IPTG (Isopropyl β-D-1-thiogalactopyranoside) and, after 13 to 15 hours, the culture was terminated when the cell concentration reached (A600 nm) OD 7 to 8.

**[TABLE 1]**

| **Division** | **Amino acid sequence** | **SEQ ID No.** |
|---|---|---|
| VHCDR1 | GYTFTSYVMH | SEQ ID NO: 1 |
| VHCDR2 | INPYNDG | SEQ ID NO: 2 |
| VHCDR3 | GAYKRGYAMDY | SEQ ID NO: 3 |
| VLCDR1 | KSSQSLFNSRTRKNYLA | SEQ ID NO: 4 |
| VLCDR2 | WASTRES | SEQ ID NO: 5 |
| VLCDR3 | KQSYYHMYT | SEQ ID NO: 6 |
| VH | EVQLQQSGPELVKPGASVKMSCKASGYTFTSYVMHWV KQKPGQGLEWIGYINPYNDGTKYNEKFKGKATLTSDK SSSTAYMELSSLTSEDSAVYYCARGAYKRGYAMDYWG QGTSVTVSSR | SEQ ID NO: 7 |
| VL | DLVMSQSPSSLAVSAGEKVTMSCKSSQSLFNSRTRKN YLAWYQQKPGQSPKLLIYWASTRESGVPDRFTGSGSG TDFTLTISSVQAEDLAVYYCKQSYYHMYTFGSGTKLE IK | SEQ ID NO: 8 |
| NVG308(scF v) | EVQLQQSGPELVKPGASVKMSCKASGYTFTSYVMHWV KQKPGQGLEWIGYINPYNDGTKYNEKFKGKATLTSDK SSSTAYMELSSLTSEDSAVYYCARGAYKRGYAMDYWG QGTSVTVSSRGGGGSGGGGSGGGGSDLVMSQSPSSLA VSAGEKVTMSCKSSQSLFNSRTRKNYLAWYQQKPGQS PKLLIYWASTRESGVPDRFTGSGSGTDFTLTISSVQA EDLAVYYCKQSYYHMYTFGSGTKLEIK | SEQ ID NO: 9 |
| NVG308 gene sequence | GAGGTCCAGCTGCAGCAGTCTGGACCTGAGCTGGTAA AGCCTGGGGCTTCAGTGAAGATGTCCTGCAAGGCTTC TGGATACACATTCACTAGCTATGTTATGCACTGGGTG AAGCAGAAGCCTGGGCAGGGCCTTGAGTGGATTGGAT ATATTAATCCTTACAATGATGGTACTAAGTACAATGA GAAGTTCAAAGGCAAGGCCACACTGACTTCAGACAAA TCCTCCAGCACAGCCTACATGGAGCTCAGCAGCCTGA CCTCTGAGGACTCTGCGGTCTATTACTGTGCAAGAGG GGCCTATAAAAGGGGATATGCTATGGACTACTGGGGT CAAGGAACCTCAGTCACCGTCTCCTCTAGAGGTGGGG GCGGTTCGGGTGGCGGGGGCTCGGGCGGGGGTGGCTC AGATCTTGTGATGTCACAGTCTCCATCCTCCCTGGCT GTGTCAGCAGGAGAGAAGGTCACTATGAGCTGCAAAT CCAGTCAGAGTCTGTTCAACAGTAGAACCCGAAAGAA CTACTTGGCTTGGTACCAGCAGAAACCAGGGCAGTCT CCTAAACTGCTGATCTACTGGGCATCCACTAGGGAAT CTGGGGTCCCTGATCGCTTCACAGGCAGTGGATCTGG GACAGATTTCACTCTCACCATCAGCAGTGTGCAGGCT GAAGACCTGGCAGTTTATTACTGCAAGCAATCTTATT ATCACATGTATACGTTCGGATCGGGGACCAAGCTGGA AATAAAA | SEQ ID NO: 10 |

### 2. Confirmation of intracellular penetration activity

According to an immunocytochemical method, a penetration ability of the antibody or fragment thereof into cells was confirmed. Specifically, Vero-E6 cells were treated with 5 µM of NVG308 and then cultured at 37 °C and under the condition of 5% CO₂ in Dulbecco's modified Eagle's medium (DMEM) + 10% FBS + 1% P/S antibiotic medium. Thereafter, the cells were stained with Alexa Fluor 488 secondary antibody and scFv antibody by immunofluorescence staining, and the intracellular penetration activity of scFv was checked for each time elapsed after treatment. Cell nuclei were stained using Antifade mounting medium with DAPI.

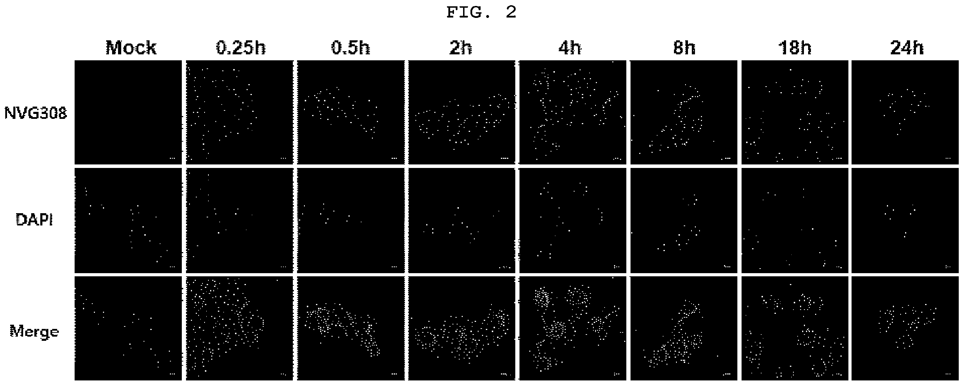

As a result, it was confirmed that scFv (labeled with green fluorescence) was present in the cells until 24 hours after protein treatment. Further, it was confirmed that the most scFv was present in the cells for 4 to 8 hours (see FIG. 2) .

### 3. Confirmation of effects of hydrolyzing viral genes

The hydrolytic activity for coronavirus spike gene (NA, HA) of the antibody or fragment thereof was confirmed. Specifically, the spike genes (NA, HA) of hCoV-OC43, PEDV and SARS-CoV-2 were synthesized using RT-PCR. The primers used for RT-PCR are shown in Table 2 below.

**[TABLE 2]**

| Division | Type of primer | Sequence | SEQ ID No. |
|---|---|---|---|
| PEDV | Forward | TGTCTCTGCGTCTTTTGGTG | SEQ ID NO: 11 |
| | Reverse | CTGCCACTTGCAGGATCATA | SEQ ID NO: 12 |
| SARS-CoV-2 | Forward | CAGATGCTGGCTTCATCAAA | SEQ ID NO: 13 |
| | Reverse | CATTGAGGCGGTCAATTTCT | SEQ ID NO: 14 |
| hCoV-OC43 Spike surface glycoprotein (S) | Forward | AGCTATACCCAATGGCAGGAA | SEQ ID NO: 15 |
| | Reverse | CCAGTGGTAATCGCTCCAC | SEQ ID NO: 16 |
| hCoV-OC43 Hemagglutinin -esterase (HE) | Forward | CGCATGTAAATGGAGATTGGTT | SEQ ID NO: 17 |
| | Reverse | CCCAAAAGAATGCCAAGCAAAA | SEQ ID NO: 18 |

1.0 µg viral gene and 0.5 µg NVG308 were mixed and reacted at 37 °C for 1 hour. As a control, 1.0 µg viral gene and 0.5 µg BSA were mixed and reacted at 37 °C for 1 hour.

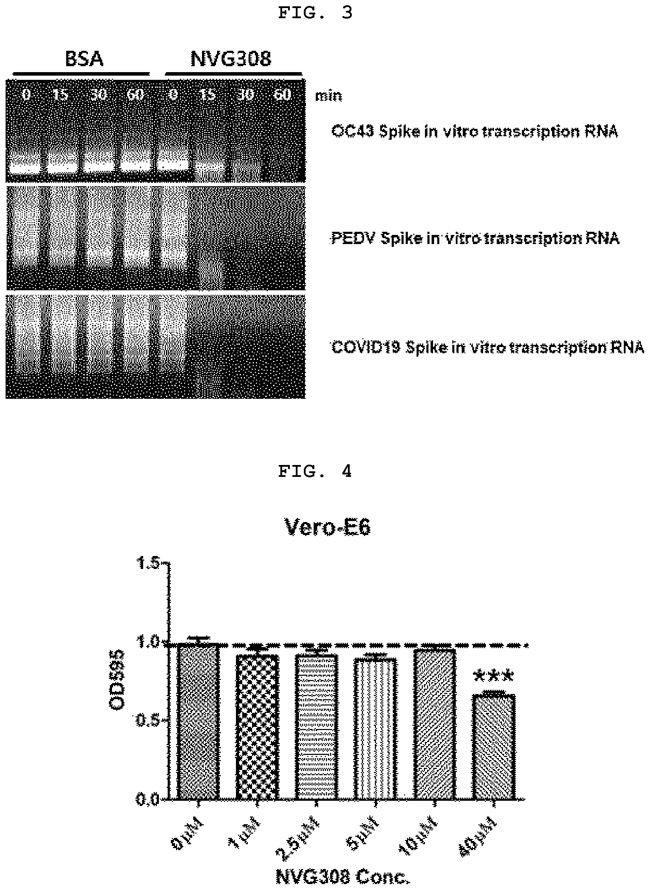

Then, as a result of confirming the nucleic acid hydrolysis ability through electrophoresis, NVG308 showed the efficacy of degrading the viral gene of the coronavirus (see FIG. 3).

### 4. Confirmation of intracellular toxicity

### 4-1. MTT analysis

To confirm the intracellular toxicity of scFv, MTT assay was performed using Vero-E6 cells. Specifically, Vero-E6 cells were treated with NVG308 at 1 µM, 2.5 µM, 5 µM, 10 µM, and 40 µM, respectively, and toxicity was confirmed after about 24 hours. As a result, there was no significant difference in cell viability up to a concentration of 10 µM. However, when treated in a concentration of 40 µM, the cell viability was reduced by about 35% (see FIG. 4).

### 4-2. NGS analysis

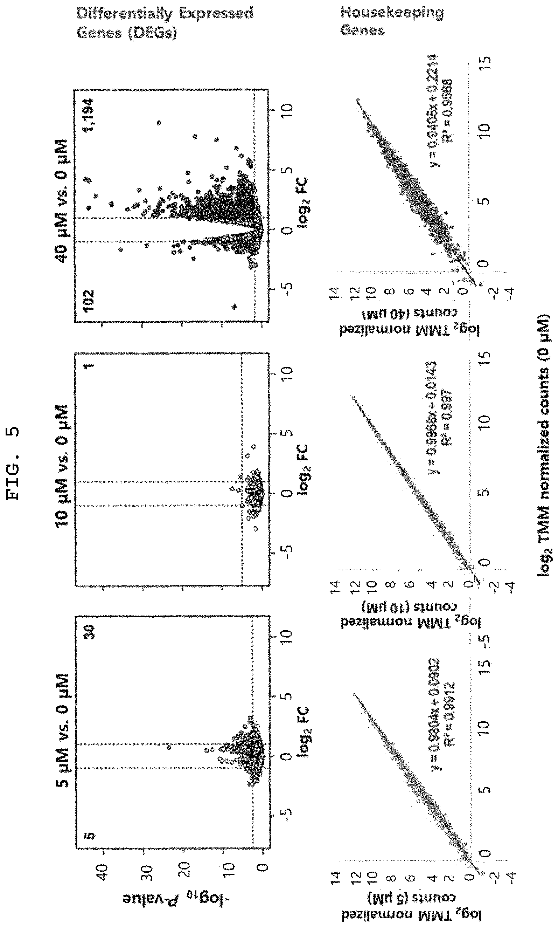

NGS analysis was performed to confirm the intracellular toxicity of scFv. Specifically, NVG308 was used for treatment of the A549 cell line in concentrations of 0 µM, 5 µM, 10 µM, and 40 µM, respectively. Genetic changes were confirmed through DEG analysis. In the groups treated with 5 µM and 10 µM of NVG308, respectively, there were few genes under up or down, whereas the groups treated with 40 µM of NVG308 showed 1,194 genes that were up and 102 genes that were down (see the upper part of FIG. 5). In addition, as a result of investigating the mutation in housekeeping genes, the R2 value was 0.9912 in the group treated with 5 µM of NVG308 and 0.997 in the group treated with 10 µM of NVG308. However, in the group treated with 40 µM of NVG308, the R2 value was 0.9588. From this result, it was confirmed that housekeeping mRNA in the group treated with 40 µM of NVG308 showed an irregular distribution (see the lower part of FIG. 5). Based on the above results, studies on antiviral efficacy were performed using 5 µM and 10 µM of NVG308.

### 5. Confirmation of antiviral efficacy against SARS-CoV-2

### 5-1. Confirmation of antiviral efficacy through qReal-time PCR

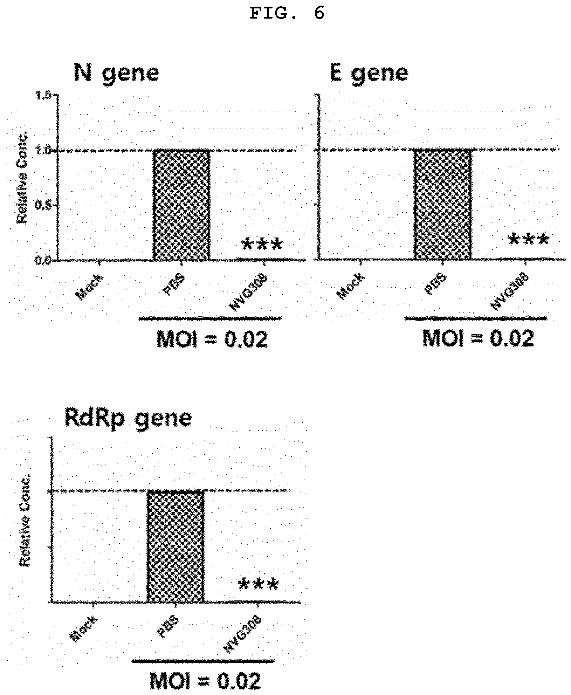

The present inventors treated the cells with NVG308 and then infected them with SARS-CoV-2 virus, followed by confirming the efficacy of inhibiting virus infection. Specifically, 24 hours after pre-treating Vero-E6 cells with NVG308 in a concentration of 5 µM, infection was implemented using SARS-CoV-2 virus at a titer of MOI of 0.02 for 48 hours. Antiviral efficacy was confirmed 24 hours after infection with the virus. Increase or decrease in N (nucleocapsid), E (envelope) and RdRp (RNA dependent RNA polymerase) virus genes of SARS-CoV-2 virus, respectively, were confirmed through gene amplification (qReal-time PCR). An amount of genes was detected by real-time qPCR. As a result, the gene amplification pattern was reduced by 99% or more in the group treated with NVG308 compared to the group treated with PBS alone for all three viral genes (see FIG. 6) .

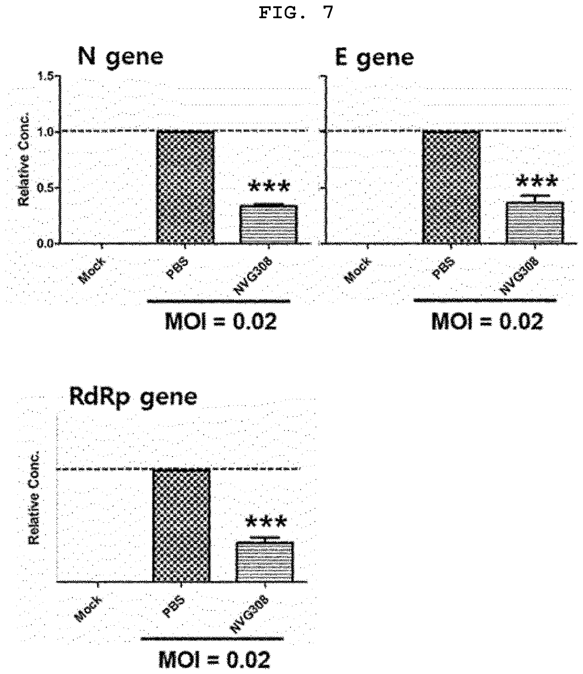

Further, the present inventors investigated the efficacy of inhibiting virus infection by treating cells with SARS-CoV-2 virus and then treating the cells with NVG308. Vero-E6 cells were infected with SARS-CoV-2 virus at a titer of MOI of 0.02 and then treated with 5 µM of NVG308 two hours later. After 24 hours of protein treatment, antiviral efficacy was confirmed. The amount of genes was detected by real-time qPCR. As a result, in the case of N gene, the viral gene amplification pattern in the NVG308-treated group was reduced by 66% compared to the PBS-only treatment group. Further, in the case of E gene, the viral gene amplification pattern in the NVG308-treated group was reduced by 63% compared to the PBS-only treatment group. Further, in the case of RdRp gene, the viral gene amplification pattern was reduced by 64% in the group treated with VG308 compared to the PBS-only treatment group (see FIG. 7).

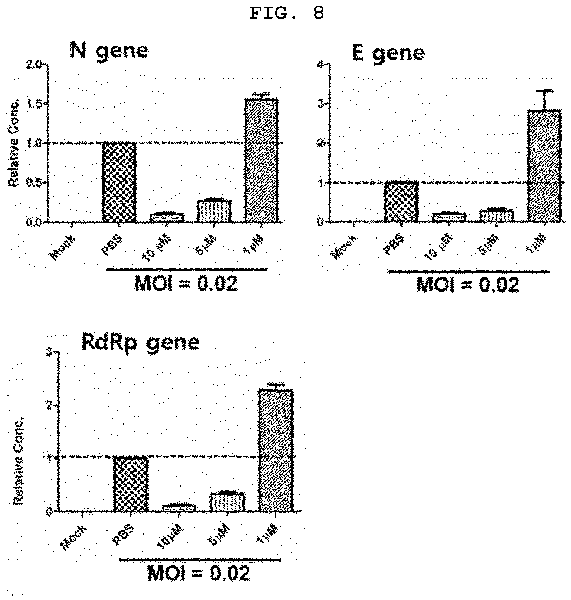

Further, after virus infection, the present inventors treated the virus by varying the concentration of NVG308 from 1 µM to 10 µM, and confirmed the antiviral efficacy against SARS-CoV-2 virus. As a result, in the group treated with 10 µM of NVG308, an expression level of N gene was decreased by about 90%, an expression level of E gene was decreased by about 81% and an expression level of RdRp gene was decreased by about 89%, respectively, compared to the PBS-treated group. Furthermore, in the group treated with 5 µM of NVG308, the expression level of N gene was decreased by about 73%, the expression level of E gene was decreased by about 73%, and the expression level of RdRp gene was decreased by about 67%, respectively, compared to the PBS-treated group (see FIG. 8).

Primers used for qPCR are shown in Table 3 below.

**[TABLE 3]**

| Virus | Target | | Sequence (5'-> 3') | SEQ ID No. |
|---|---|---|---|---|
| SARS-CoV-2 | RdRP gene | Forward | GTG ARA TGG TCA TGT GTG GCG G | SEQ ID NO: 19 |
| | | Reverse | CAR ATG TTA AAS ACA CTA TTA GCA TA | SEQ ID NO: 20 |
| | E gene | Forward | ACA GGT ACG TTA ATA GTT AAT AGC GT | SEQ ID NO: 21 |
| | | Reverse | ATA TTG CAG CAG TAC GCA CAC A | SEQ ID NO: 22 |
| | N gene | Forward | CAC ATT GGC ACC CGC AAT C | SEQ ID NO: 23 |
| | | Reverse | CAG GAA CGA GAA GAG GCT TG | SEQ ID NO: 24 |

### 5-2. Confirmation of antiviral efficacy through plaque assay

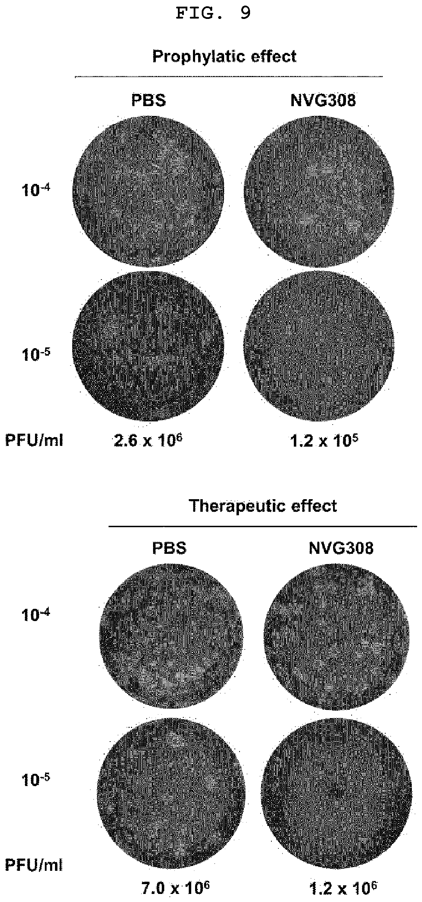

From both of a sample infected with SARS-CoV-2 virus after treating Vero-E6 cells with 10 µM of NVG308 by the method described in 5-1 above, and another sample treated with 10 µM of NVG308 after infecting Vero-E6 cells with SARS-CoV-2 virus, soups of the culture medium were obtained. The number of viruses was measured by performing a plaque assay with each obtained soup. As a result, the group infected with SARS-CoV-2 virus after treatment with PBS showed 2.6 × 10⁶ PFU per 1 ml, while the group infected with SARS-CoV-2 virus after treatment with 10 µM of NVG308 showed 1.2 × 10⁵ PFU per 1 ml. That is, even when NVG308 was treated before virus infection, it could be confirmed that the number of SARS-CoV-2 viruses was significantly reduced compared to the untreated group (see the upper part of FIG. 9). Further, the group treated with only PBS after infection with SARS-CoV-2 virus showed 7.0 × 10⁶ PFU per 1 ml, while the group treated with 10 µM of NVG308 after infection with SARS-CoV-2 virus showed 1.2 × 10⁶ PFU per 1 ml. That is, even when NVG308 was treated after virus infection, it could be confirmed that the number of SARS-CoV-2 viruses was significantly reduced compared to the untreated group (see the lower part of FIG. 9).

### 6. Confirmation of antiviral efficacy of antibody or fragment thereof against hCoV-OC43 and PEDV

### 6-1. Confirmation of antiviral efficacy through qReal-time PCR

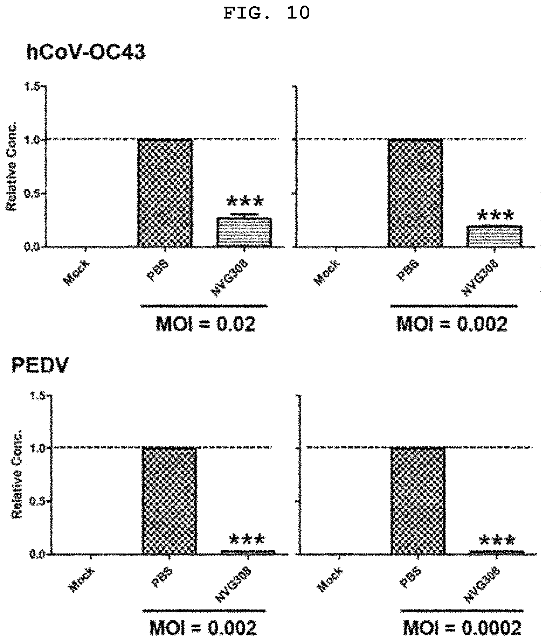

After infecting hCoV-OC43 virus, the present inventors confirmed the efficacy of inhibiting virus infection by NVG308 treatment. Specifically, after infecting Vero-E6 cells with hCoV-OC43 virus at titers of MOI of 0.02 and 0.002, the cells were treated with 5 µM of NVG308 two hours later, followed by measuring antiviral efficacy 48 hours later. An amount of gene was detected by real-time gene amplification (Real-time qPCR). As a result, compared to the group treated with only PBS, the group treated with hCoV-OC43 at MOI of 0.02 had a decrease of about 73% in N gene, while it was confirmed that the group treated with hCoV-OC43 at MOI of 0.002 showed a decrease of about 81% in N gene (see the upper part of FIG. 10).

Further, the present inventors confirmed the efficacy of inhibiting virus infection by treating NVG308 after infecting the PEDV virus. Specifically, after infecting Vero cells with PEDV virus at titers of MOI of 0.002 and 0.0002, respectively, the cells were treated with 5 µM of NVG308 two hours later, followed by measuring antiviral efficacy 24 hours later. An amount of gene was detected by real-time qPCR. As a result, compared to the PBS-only treatment group, both the groups treated with PEDV at MOI of 0.002 and 0.0002, respectively, showed a decrease of about 97% in N gene (see the lower part of FIG. 10).

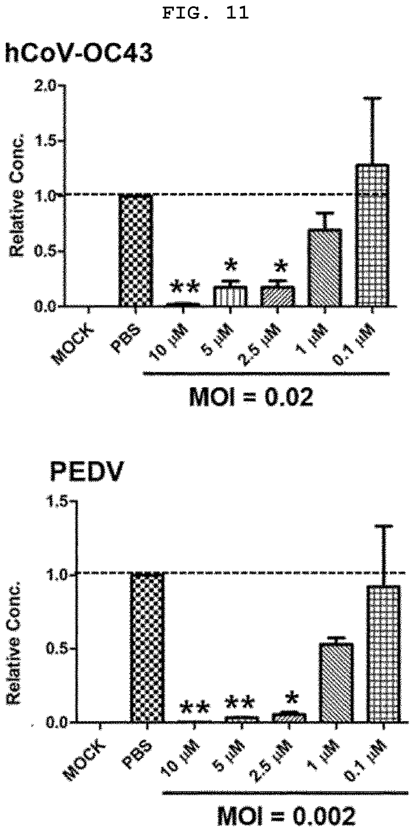

Further, the present inventors treated cells with NVG308 while varying the concentration of NVG308 from 0.1 µM to 10 µM after virus infection, and then measured the antiviral efficacy for each virus. As a result, in the case of hCoV-OC43 virus, the antiviral efficacy was confirmed in the groups treated with 2.5 µM, 5 µM and 10 µM of NVG308, respectively, and the groups showed effects of decreasing N genes by 82%, 82%, and 98%, respectively, compared to PBS-treated group. In the case of PEDV virus, the antiviral efficacy was confirmed in the groups treated with NVG308 at 2.5 µM, 5 µM and 10 µM, respectively. Specifically, compared to the PBS-treated group, the above groups exhibited each N-gene reducing effect of 95%, 97% and 99%, respectively (see FIGS. 11).

Primers used for qPCR are shown in Table 4 below.

**[TABLE 4]**

| Viru s | Target | | Sequence (5'-> 3') | SEQ ID No. |
|---|---|---|---|---|
| HCoV-OC43 | N gene | Forward | AGT ACT GCT ATT TCT TGT TCA AAC ATG TG | SEQ ID NO: 25 |
| | | Reverse | GCT CTA CGA CTT CGT CTG TTT TTA GA | SEQ ID NO: 26 |
| PEDV | N gene | Forward | GAT CGT GGC CGC AAA CG | SEQ ID NO: 27 |
| | | Reverse | TGC CAT TGC CAC GAC TCC | SEQ ID NO: 28 |

### 6-2. Confirmation of antiviral efficacy through plaque assay

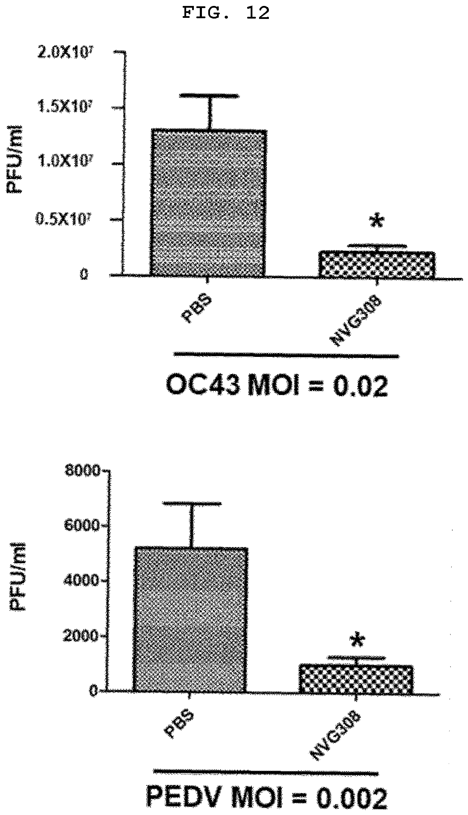

After infecting Vero-E6 cells with the hCoV-OC43 virus at MOI of 0.02 by the method described in 6-1 above, a soup of culture medium obtained from a sample treated with 10 µM of NVG308 was prepared. The number of viruses was measured by performing a plaque assay with the prepared soup. As a result, the PBS-treated group showed 1.3 × 10⁷ PFU per 1 ml, while the NVG308-treated group showed 2.2 × 10⁶ PFU per 1 ml. That is, it could be confirmed that the number of hCoV-OC43 viruses was reduced by 82% through NVG308 treatment (see the upper part of FIG. 12).

Further, after infecting Vero cells with PEDV virus at an MOI of 0.002, plaque assay was performed using a soup of culture medium obtained from a sample treated with 10 µM of NVG308. As a result, the group treated with only PBS showed 5.2 × 10³ PFU per 1 ml, while the group treated with NVG308 showed 1.0 × 10³ PFU. That is, it could be confirmed that the number of PEDV viruses was reduced by 81% through NVG308 treatment (see the lower part of FIG. 12).

### 6-3. Confirmation of antiviral efficacy by immunoblot

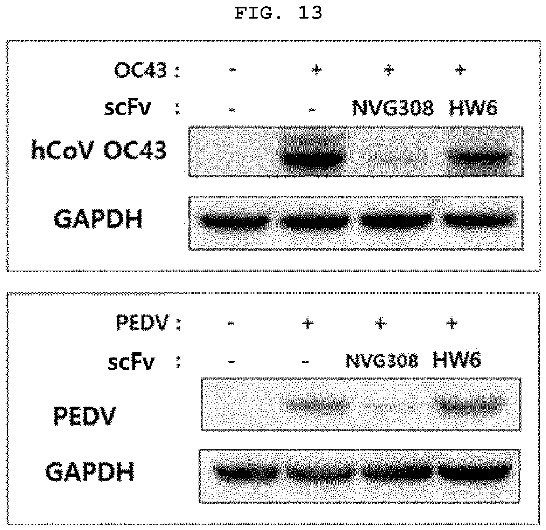

The change in amount of protein in each virus was determined using an antibody against the spike protein of hCoV-OC43 and PEDV virus. Specifically, in the case of hCoV OC43, Vero E6 cells were infected with hCoV-OC43 virus at MOI of 0.02, followed by treatment using 5 µM of NVG308. Herein, HW6, which is scFv without nucleolytic activity, was used as a negative control. After culturing for 48 hours, Western blotting was performed to detect an amount of virus spike protein. As a result, it was confirmed that the amount of spike protein was significantly reduced in the experimental group treated with scFv compared to the experimental group infected with only the virus. On the other hand, it was confirmed that the experimental group treated with HW6 showed very little decrease in amount of the spike protein. In addition, after infecting Vero cells with PEDV virus at MOI of 0.001, followed by treatment using 5 µM of NVG308. Then, after culturing for 48 hours, Western blotting was performed to detect the amount of virus spike protein. As a result, in the case of PEDV, it was also confirmed that the amount of spike protein was significantly reduced in the experimental group treated with NVG308 compared to the experimental group infected with only the virus (see FIG. 13).

Through the above experimental results, the antibody or fragment thereof according to the present invention did not show toxicity to cells in a concentration of 10 µM or less, instead, it was confirmed that antiviral efficacy against various coronaviruses (SARS-CoV-2, hCoV-OC43, and PEDV) could be achieved.

The description of the present invention is for illustrative purposes, and those skilled in the art to which the present invention pertains will understand that the present invention can be easily modified into other specific forms without changing the technical spirit or essential features of the present invention. Accordingly, it should be understood that the embodiments described above are illustrative in all respects and not restrictive.

The scope of the present invention is indicated by the following appended claims, and all changes or modifications derived from the meanings and scope of the claims and their equivalents should be construed as being included in the scope of the present invention.

## Claims

1. An antiviral composition for coronavirus, comprising an antibody or fragment thereof, which includes: a heavy chain CDR1 (VH CDR1) represented by SEQ ID NO: 1; VH CDR2 represented by SEQ ID NO: 2; VH CDR3 represented by SEQ ID NO: 3; a light chain CDR1 (VL CDR1) represented by SEQ ID NO: 4; VL CDR2 represented by SEQ ID NO: 5; and VL CDR3 represented by SEQ ID NO: 6.

2. The antiviral composition according to claim 1, wherein the antibody or fragment thereof includes a heavy chain variable region comprising an amino acid sequence of SEQ ID NO: 7.

3. The antiviral composition according to claim 1, wherein the antibody or fragment thereof includes a light chain variable region comprising an amino acid sequence of SEQ ID NO: 8.

4. The antiviral composition according to claim 1, wherein the fragment is at least one selected from the group consisting of single-chain antibodies, Fv, scFv, di-scFv, Fab, Fab', F(ab')₂ and diabody.

5. The antiviral composition according to claim 1, wherein the fragment is scFv comprising an amino acid sequence of SEQ ID NO: 9.

6. The antiviral composition according to claim 1, wherein the antibody or fragment thereof is a humanized antibody or fragment thereof; or a chimeric antibody or fragment thereof.

7. The antiviral composition according to claim 1, wherein the coronavirus is at least one selected from the group consisting of genus alpha-coronavirus or genus beta-coronavirus.

8. The antiviral composition according to claim 1, wherein the coronavirus is at least one selected from the group consisting of SARS-coronavirus-2 (SARS-CoV-2), human coronavirus OC43 (hCoV-OC43) and pig epidemic diarrhea virus (PEDV).

9. A pharmaceutical composition for prevention or treatment of a coronavirus-infectious disease, comprising the composition of any one of claims 1 to 8.

10. The pharmaceutical composition according to claim 9, wherein the coronavirus is at least one selected from the group consisting of SARS-coronavirus-2 (SARS-CoV-2), human coronavirus OC43 (hCoV-OC43) and pig epidemic diarrhea virus (PEDV).

11. A food composition for prevention or treatment of coronavirus-infectious diseases, comprising the composition of any one of claims 1 to 8.

12. The food composition according to claim 11, wherein the coronavirus is at least one selected from the group consisting of SARS-coronavirus-2 (SARS-CoV-2), human coronavirus OC43 (hCoV-OC43) and pig epidemic diarrhea virus (PEDV).
